# EUROPEAN PATENT SPECIFICATION

(11) **EP 0 943 291 B1**
(45) Date of publication and mention of the grant of the patent: **14.04.2004**
(21) Application number: 99830088.3
(22) Date of filing: 18.02.1999
(51) Int. Cl.: A61B 6/04

(54) **Tiltable patient support table, in particular for X-ray observations**
Schwenkbarer Patiententisch, insbesondere für Röntgenuntersuchungen
Support pivotable pour patient, destiné particulièrement à l'examen radiologique

(30) Priority: 11.03.1998 IT MI980490
(43) Date of publication of application: 22.09.1999
(73) Proprietor: Mecall S.r.l., 20035 Lissone (MI) (IT)
(72) Inventor: Verpelli, Sergio, 20053 Muggiò MI (IT)
(74) Representative: Adorno, Silvano

(56) References cited:
- EP-A- 0 511 049
- DE-A- 4 415 503
- GB-A- 893 770
- US-A- 3 441 266
- US-A- 3 805 080
- US-A- 4 131 801

## Description

A tiltable patient support table is described, in particular for X-ray observations, but possibly usable for whichever diagnostic examination or also therapeutic treatment for which the patient, suitably secured to the table itself, can assume whichever inclined position with respect to the horizontal and even opposite vertical positions at 180 degrees from each other.

Tiltable tables are known, usually operated by means of a remote control, which will be called for "X-ray" use, although their application could be different, as mentioned above. These tables are normally associated with the indication 90/90 just due to their characteristic of allowing a rotation of ± 90°, thus along 180° in all, starting from the horizontal position. It is also clear that, since a table structure of this type has a length that can reach a size of 2300 mm, while the lowest height from the floor must be such as to allow an access as easy as possible also to patients being aged or having some physical handicaps, whereby comprised between 780 and 820 mm, it should be absolutely avoided that one of the table ends may collide with the floor as soon as the rotation starts. It should also be appreciated that under the patient support table there is usually mounted an electronic device, which is called brilliance intensifier (shortly B.I.), in order to improve the performance of the apparatus when obtaining electronic images with a small amount of X-rays to the patient. When this device is placed at the table end that, upon starting, approaches the floor, its volume would in practice cause immediately a collision at the beginning of the rotation, if there is no provision about the possibility of raising the rotational fulcrum of the tiltable table before and during its rotation.

Since some decades it is already known that such a problem is simply solved by mounting the rotational fulcrum of the tiltable structure to a vertically sliding element moved e.g. by a screw driven by a motor, generally through the intermediary of a transmission capable of reducing the number of revolutions of the motor and rendering it adequate to the speed of upward movements of the slider. In this respect reference should be made e.g. to the U.S. Patent 4.912.754 and European patent No. 0612501, in addition to several embodiments of tiltable patient support tables manufactured and put on the market by the applicant and many other manufacturers active in this field among which Philips and Siemens could be cited.

The problem of ensuring the possibility of rotation of a patient support table along an angle of at least ± 90° with contemporary lifting of the same to avoid collisions with the floor or anyhow in order that its height can be controlled also at a horizontal position, has been therefore solved until now in a satisfactory way, but two important dependent problems remain unsolved owing to the weight and the overall size of the apparatus. As a matter of fact it should be appreciated that the so-called vertical slider with varying height shall bear as a cantilever the patient support table as such with its load and support structure, the above-mentioned B.I. device, the seriograph having the function of capturing radiographic images on a film, the slidable tower carrying the X-ray tube with collimator, etc., all for a total weight in the order of 1000 kg. As a consequence both the vertical slider and the frame in which it slides should be rather solid, and consequently also heavy. This involves non negligible inconveniences when the patient support table has to be installed at the upper floors of a building, but also in general owing to the fact that the bigger quantity of material employed results unavoidably in a higher cost of the apparatus. On the other hand the vertical structure having the function of lifting the rotational fulcrum is necessarily very encumbering as to the depth size in all the known embodiments in which the means bearing as a cantilever the weight of the patient support table and its accessories is rotatably mounted on the vertical slider. This involves the drawback of rendering extremely difficult and even impossible the installation in relatively small rooms, while also limiting the accessibility of the operators around the patient being examined.

Therefore it is an object of the present invention to provide a tiltable patient support table of the above-mentioned type which does not show these inconveniences, typical of the prior art, by resolving of directly transmitting to the floor the weight of the table and its accessories which previously was cantilevered and now thereby can be reduced, thus there being no longer the need of opposing the tilting torque in the vertical plane which is just caused by the cantilevered mounting. Furthermore, also the encumbrance of the depth size is reduced, thanks to the fact that the table supporting means is mainly located thereunder, thus remaining within the limits of the table transverse size without the need of additional external space to be occupied.

EP-A-0 511 049 discloses a tiltable patient support table according to the preamble of claim 1, which solves some of the problems associated with known cantilevered structures. It is an object of the present invention to provide an alternative to the construction disclosed in EP-A-0 511 049 which also remedies to the problems associated with cantilevered constructions.

These objects are achieved by means of a tiltable patient support table showing the features of claim 1.

These and additional objects, advantages and features of the present invention will be clearer from the following detailed description of a preferred embodiment thereof, given by way of a non-limiting example with reference to the annexed drawings in which:

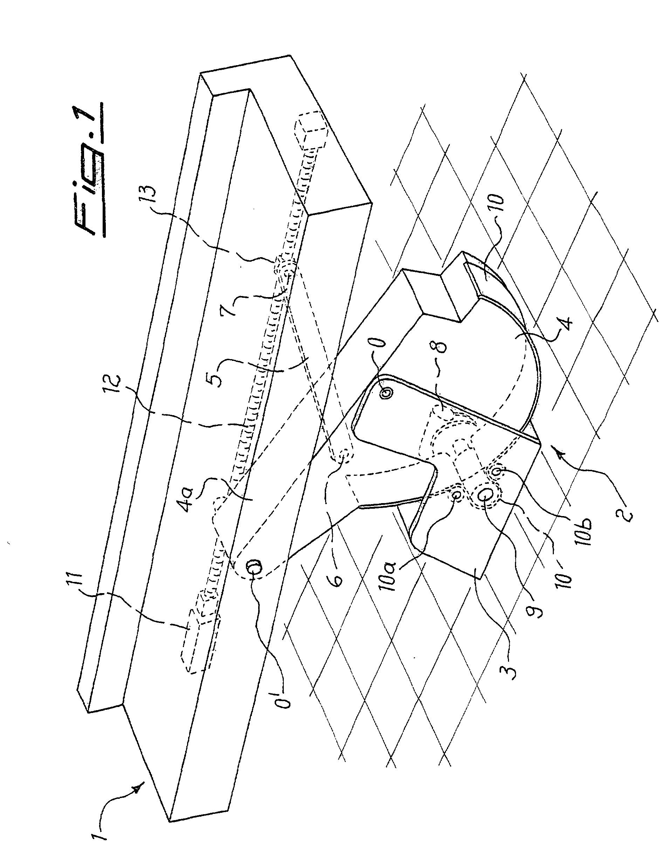

**Figure 1** shows a schematic, perspective view of the X-ray table according to the present invention;
**Figure 2** shows a front view, in the direction of the greater size, of the table of figure 1 in a lowered position;
**Figure 3** shows a view similar to that of figure 2 with the table in a vertical position upon a rotation of 90° in clockwise direction;
**Figure 4** shows again the same table in a horizontal position, raised with respect to that of figure 2; and
**Figure 5** shows the vertical position, rotated by 180° with respect to the position of figure 3, obtained by e.g. passing through an intermediate position like in figure 4, at the end of a counterclockwise rotation of the same table.

With reference to the drawings, it appears that the apparatus of the present invention is substantially composed of two portions, namely an upper part indicated as 1, comprising the actual beam or table suitable to support the patient, as well as all those mechanical,optical,radiological and electric/electronic devices useful to the operation of the apparatus, and a lower portion 2, fixed to the floor, suitable to support the real table, and comprising the movement means causing its rotation and lifting.

Said portion 2 comprises a base 3 fixed to the apparatus rest floor, to which base there is rotationally mounted, in its center of rotation O, a sector of circle 4, formed e.g. as a big, substantially semi-circular plate, being hinged in the geometrical center O of the arc of circle which forms part of its profile, by means of a pivot mounted at its ends on the base 3, possibly having a fork-like shape, at the inside of which said sector 4 can rotate by an angle of about 90°. The same sector shows, on its side directed toward the center portion of table 1, an extension or arm 4a pivotedly mounted, near its end, to the structure of the table itself by means of a pivot O'. Therefore the sector 4 is hinged, at two different pivotal points O, O' rather spaced apart from each other, respectively to the base 3 and to the patient support table 1. Suitable motor means causes the rotation of sector 4 with respect to base 3, while other motor means, being integral to the structure itself of table 1, bring about the movement in the longitudinal direction of a pivotal end 7 of connecting rod or strut 5, the other end of which is hinged to the sector 4 at a point 6 intermediate between pivots O and O'.

In a preferred embodiment, with reference to the drawings, the first motor means are formed as a reduction gear 8 being integral with the base 3 and driving a pinion 9 which meshes with a toothed chain or belt 10 also winding up, by means of two guide pulleys 10a and 10b, onto the arc-shaped periphery of the sector 4 to which both its ends are fixed.

The second motor means determining the rotation of table 1 with respect to the second pivot O' are preferably formed as a second reducing gear 11 mounted to the tilting table structure 1 for the rotation of a screw 12 which longitudinally extends along said structure, being engaged in a lead screw 13 carrying the pivoting end 7 of the strut 5. Thereby a movement of lead screw 13 in consequence of the rotation of screw 12 gives rise to a variation of the distance between the pivotal points 7 and O' with contemporary counterclockwise rotation of the strut 5 around hinge 6, and consequently a necessary clockwise rotation, as shown in the drawings, of the structure 1 with respect to pivot O'.

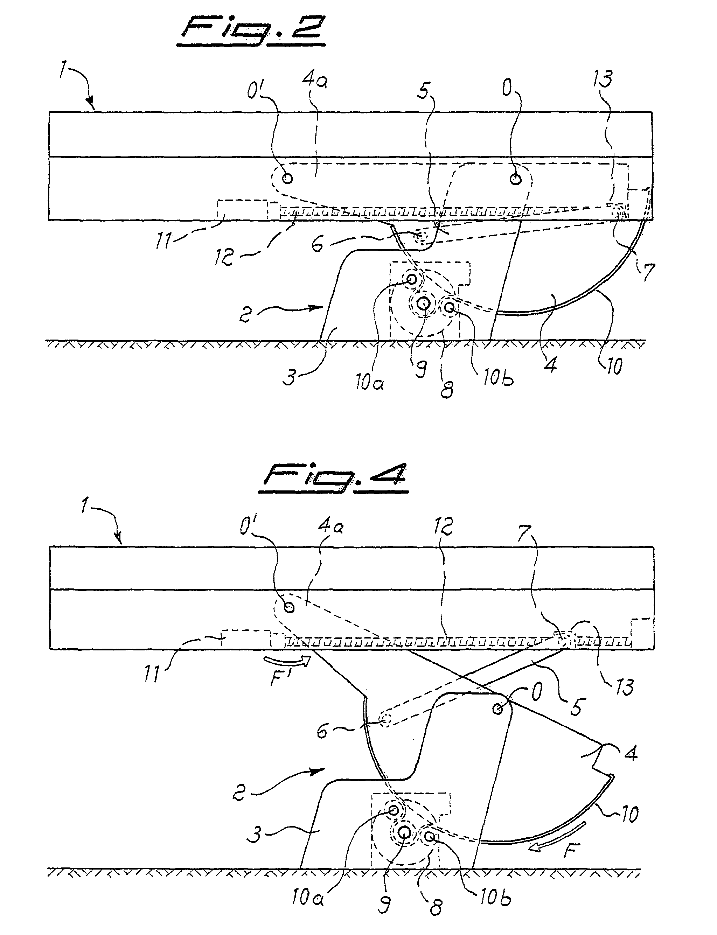

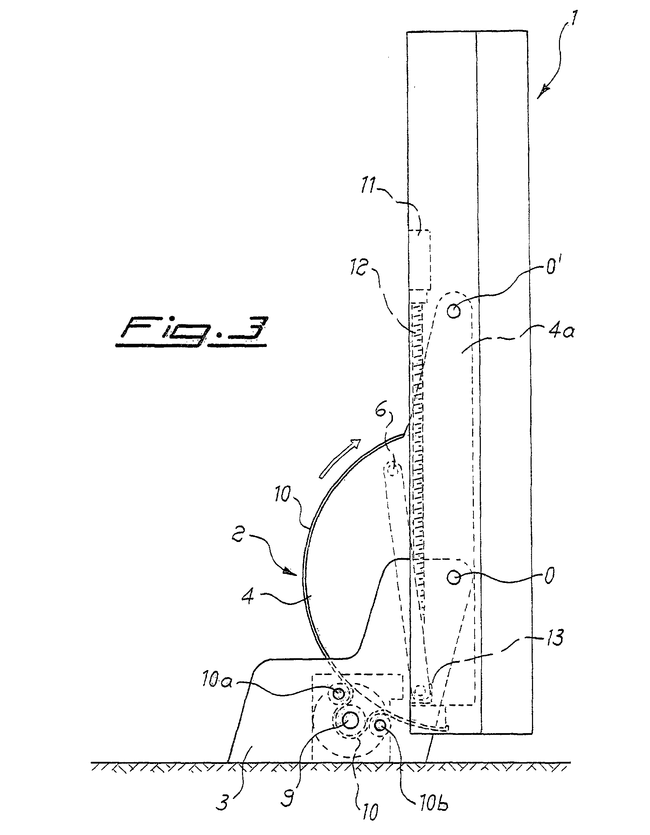

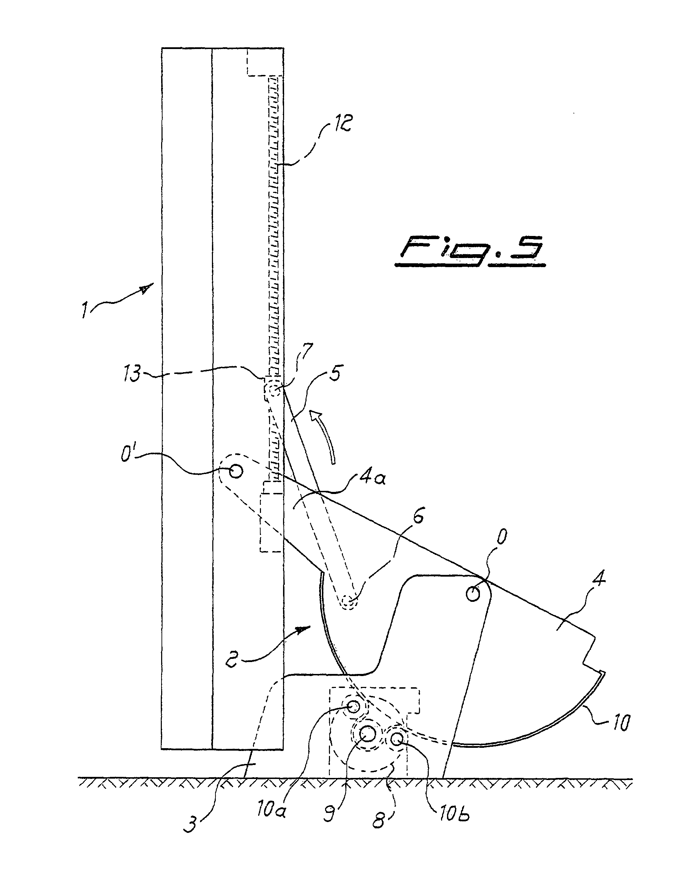

Referring in particular to figures 2-5 and starting from the initial lowered position, that we could define "rest" position, of figure 2, some typical positions are represented, such as the clockwise rotation of the structure until reaching the vertical (Figure 3).

Such a position is achieved by only operating the motor gear 8 the movement of which, through the toothed chain or belt 10, causes the table structure 1 with its load to rotate about pivot O in a clockwise direction as represented in the drawings, according to arrow F, while the motor gear 11 is kept stationary and the strut 5 is not subject to any action.

Figure 4 shows a position of table 1 which is upraised with respect to figure 2 in consequence of a simple vertical movement of the structure while the patient is kept parallel to the floor. Starting from the position of figure 2, the two reduction gears 8 and 11 are caused to rotate at the same time according to a suitable law controlling the relative movement of one with respect to the other, in particular of motor gear 11 on the structure 1 with respect to the one on base 3. The resulting movement shall be in fact the desired one, that is a vertical movement without table tilting with respect to the floor. The synchronization of the two movements, giving rise to rotations in two opposite directions, respectively clockwise according to arrow F about pivot O and counterclockwise according to arrow F' about pivot O', is determined trough well known electronic or mechanical coupling means of the two motors. It should also be appreciated that the position of figure 4 could be reached as an intermediate position between that of figure 3 and the one, tilted by 180° thereto, as shown in figure 5. In this case the rotation of sector 4 about the pivot O will be of opposite direction, i.e. counterclockwise, with respect to the one indicated in figure 4 by the arrow F.

Finally, with reference to figure 5, representing the so-called "Trendelenburg" position, corresponding to the patient oriented with his/her head down, assuming that in the horizontal position of Figures 2 and 4 his/her head orientation is towards the left side of the drawings, such a position is obtained in two steps, the first of which is performed by causing the table structure to be lifted until an adequate height, for example as already stated before with reference to figure 4. Subsequently the motorized reducing gear 8 is stopped and the reducing gear 11 continues its operation so as to bring the lead screw 13, and therefore pivoting point 7, near to the fulcrum O' about which the structure 1 is forced to rotate in counterclockwise direction according to arrow F', driven by strut 5 during its rotation around hinge 6. It is thus possible to reach a position which is overturned to 180° with respect to that represented in figure 3.

Although not represented in the drawings, there is suitably provided a guide with circulation of balls for the screw 12 and the screw itself may be of the circulating balls type to reduce the friction as much as possible, taking into account the total weight to move, of about 1500 Kg. Furthermore, as it is known, there will be provided remote control means for a long-distance actuation of the motor reducing gears 8 and 11 as well as some detecting means in the proximity of the apparatus to avoid any possible incidental collision during the movement, even against unforeseen obstacles, either stationary or mobile, while the above-mentioned law for the mutual coupling of the two motor reducing gears in the standard movements for avoiding the impact with the floor can already be preset in the software of a programmed logic processor controlling the whole process.

Different positions from those shown in the drawings could be provided for the reducing gear 8 and 11, corresponding of course to different rotational directions about pivots O and O', such as to allow in any case whichever angle of inclination of the tiltable table until the two opposite vertical positions at 180° to each other.

## Claims

1. A tiltable patient support table, in particular for X-ray observations, comprising a table structure (1) and support means (2) for bearing said structure by imparting to the same movements of rotation and translation in the vertical plane, wherein said support means (2) comprises a stationary base (3) essentially underlying the structure (1) and suitable to transmit the weight thereof to the rest floor, wherein said base (3) is rotatably mounted around a pivot axis (O), transverse with respect to said table structure (1), and wherein a support element (4) is in turn hinged to the table structure (1) in a second pivot (O') with axis parallel to said first pivot (O), said table structure (1) being linked to said support element (4) also through a connecting rod or strut (5) hinged thereto; the tiltable patient support table being **characterised in that** an end (6) of the connecting rod or strut (5) ends at a point comprised between the two pivots (O, O') and the other end at a point (7) whose position is variable along said structure, there being further provided means (8) to control the rotation of said support element (4) about pivot (O) and second means (11) to control the longitudinal movement of said pivoting point (7) along said structure (1).

2. A patient support table according to claim 1, **characterized in that** said element (4) is comprised of a toothed arc of circle sector with the center corresponding to first pivot axis (O) meshing with elements (9, 10) driven by a motor reducing gear (8) and having an extension or arm (4a) with said second pivot (O') in its end zone most distant from the center (O).

3. A patient support table according to claim 1 or 2, **characterized in that** said second control means (11) are provided by a motor reducing gear suitable to cause the rotation, about its axis integral with the table structure, of a rotatable screw (12) extending longitudinally to the direction of the greater size of the table itself, said pivoting point (7) of strut (5) to the table structure (1) being provided integral with a lead screw (13) meshing with the rotatable screw (12).

4. A patient support table according to claim 2, **characterized in that** said base (3) is shaped as a fork or cradle with two outer mountings between which the support element (4) is housed, whereby it can rotate about an axis passing through its geometrical center (O).

5. A patient support table according to claims 2 or 4, **characterized in that** on the peripheral portion of the arc of circle sector (4) there is wound up a toothed chain or belt (10) which is fixed at its both ends to said sector and is partially meshing with a pinion (9) which is driven by said reducing gear (8), there being provided means (10a, 10b) to ensure that the path of the toothed chain or belt (10) is closed as strictly as possible onto the periphery of sector (4) at both sides of pinion (9).

6. A patient support table according to claims 2 and 3, **characterized by** comprising means of automatic mutual coupling between the two reducing gears (8, 11) for arranging their contemporary operation giving rise to opposite direction rotations of sector (4) about the pivot (O) and of the structure (1) about pivot (O'), whereby the resulting movement of the said structure is a translation in the vertical plane without inclinations with respect to the horizontal.

## Patentansprüche

1. Kippbarer Krankhaltetisch, besonders für Röntgenuntersuchungen, enthaltend ein Tischgefüge (1) und Lagermittel (2) zur Unterstützung des genannten Gefüges durch Erteilung von Drehungs- und Translationsbewegungen in die Vertikalebene, wobei das genannte Lagermittel (2) einen standfesten, wesentlich unter dem Gefüge (1) liegenden Fuss (3) enthält, der das Gewicht des Gefüges dem Auflageboden zu Übertragen geeignet ist, wobei der genannte Fuss (3) einen Achse (O) herum, quer zu dem genannten Gefüge (1) schwenkbar montiert ist, und wobei ein Tragelement (4) am Tischgefüge (1) in einem zweiten achsparallelen zum ersten Achse (O) Drehachse (O') seinerseits angelenkt ist, wobei das genannte Tischgefüge (1) mit dem genannten Tragelement (4) auch durch eine an diesen angelenkte Anschlusspleuelstange oder Strebe (5) verbunden ist, **dadurch gekennzeichnet, dass** ein Ende (6) der Anschlusspleuelstange oder Strebe (5) in einem zwischen beiden Drehachsen (O, O') angeordneten Punkt und das andere Ende in einem Punkt (7) der lageveränderlich an dem genannten Gefüge entlang ist beenden, wobei Mittel (8) um die Schwenkung des genannten Tragelements (4) der Achse (O) herum zu steuern sowie zweite Mittel (11) um die longitudinale Bewegung des genannten schwenkbaren Punktes (7) an dem genannten Gefüge (1) zu steuern vorgesehen sind.

2. Kippbarer Krankhaltetisch gemäss Anspruch 1, **dadurch gekennzeichnet, dass** das genannte Element (4) aus einem kreisbogenförmigen gezackten Sektor besteht dessen Zentrum mit der ersten Drehachse (O) übereinstimmt und der in durch einen Getriebemotor (8) betätigten Elementen (9, 10) eingreift und der einen Ansatz oder Arm (4a) hat, wobei sich die genannte zweite Drehachse (O') in seiner weitest stehenden von Zentrum (O) Zone befindet.

3. Kippbarer Krankhaltetisch gemäss Anspruch 1 oder 2, **dadurch gekennzeichnet, dass** die genannten zweiten Steuermittel (11) aus einem Getriebemotor bestehen, der eine sich nach der Länge der grösseren Abmessung des Tischgefüges ausweitende drehbare Schraube (12) um seiner am Tischgefüge befestigten Achse zu drehen geeignet ist, wobei der genannte Anlenkpunkt (7) der Strebe (5) am Tischgefüge (1) einteilig mit einer mit der drehbaren Schraube (12) eingreifenden Muttergewinde (13) vorgesehen ist.

4. Kippbarer Krankhaltetisch gemäss Anspruch 2, **dadurch gekennzeichnet, dass** der genannte Fuss (3) gabel- oder wiegeweise geformt ist, mit zwei zwischen sich das Tragelement (4) aufnehmenden Aussenständern, damit es um sein geometrisches Zentrum (O) drehen kann.

5. Kippbarer Krankhaltetisch gemäss Anspruch 2 oder 4, **dadurch gekennzeichnet, dass** auf dem Randteil des kreisbogenförmigen Sektors (4) eine Kette oder ein Zahnriemen aufgewickelt ist, die (der) mit seinen Enden auf dem genannten Sektor befestigt und teilweise auf einen Ritzel (9) aufgewickelt ist, das von dem genannten Getriebemotor (8) betätigt ist, wobei Glieder (10a, 10b) vorgesehen sind, um die höchste Zusammenziehung der Strecke der Kette oder des Zahnriemens (10) auf dem Umfang des Sektors (4) an beiden Seiten des Zahnriemens (9) zu sichern.

6. Kippbarer Krankhaltetisch gemäss Ansprüchen 2 und 3, **dadurch gekennzeichnet, dass** er automatische Verriegelungsmittel zwischen beiden Getriebemotoren (8, 11) zur Vorbereitung ihrer gleichzeitiger Betätigung enthält, um gegenseitige Drehungen des Sektors (4) um die Achse (O) und des Gefüges (1) um die Drehachse (O') herum zu bewirken, so dass die resultierende Bewegung des genannten Gefüges eine Verschiebung in der senkrechten Ebene ohne Neigungen in Bezug auf die Horizontale ist.

## Revendications

1. Table de support de patient inclinable, en particulier pour des examens radiologiques, comportant une structure de table (1) et des moyens de support (2) pour supporter ladite structure en communiquant les mêmes déplacements de rotation et de translation dans le plan vertical, lesdits moyens de support (2) comportant une base stationnaire (3) essentiellement sous-jacente à la structure (1), et adaptée pour transmettre le poids de celle-ci sur le sol d'appui, ladite base (3) étant montée de manière rotative autour d'un axe de pivotement (O), transversalement par rapport à ladite structure de table (1), et un élément de support (4) étant à son tour articulé sur la structure de table (1) dans un second pivot (O') ayant un axe parallèle audit premier pivot (O), ladite structure de table (1) étant reliée audit élément de support (4) également à travers une tige de connexion ou entretoise (5) articulé sur celle-ci, la table de support de patient inclinable étant **caractérisée en ce qu'**une première extrémité (6) de la tige de connexion ou entretoise (5) se termine au niveau d'un point compris entre les deux pivots (O, O'), et l'autre extrémité au niveau d'un point (7) dont la position est variable le long de ladite structure, des premiers moyens (8) pour commander la rotation dudit élément de support (4) autour du pivot (O) et des seconds moyens (11) pour commander le déplacement longitudinal dudit point de pivotement (7) le long de ladite structure (1) étant de plus fournis.

2. Table de support de patient selon la revendication 1, **caractérisée en ce que** ledit élément (4) est constitué d'un secteur en arc de cercle muni de dents, le centre correspondant au premier axe de pivotement (O) engrenant avec des éléments (9, 10) entraînés par un engrenage réducteur de moteur (8), et ayant un prolongement ou bras (4a) avec ledit second pivot (O') dans sa zone d'extrémité la plus éloignée du centre (O).

3. Table de support de patient selon la revendication 1 ou 2, **caractérisée en ce que** lesdits seconds moyens de commande (11) sont fournis par un engrenage réducteur de moteur adapté pour provoquer la rotation, autour de son axe en un seul bloc avec la structure de table, d'une vis rotative (12) s'étendant longitudinalement par rapport à la direction de la dimension la plus importante de la table elle-même, ledit point de pivotement (7) de l'entretoise (5) de la structure de table (1) étant agencé en un seul bloc avec une vis mère ( 13) engrenant avec la vis rotative (12).

4. Table de support de patient selon la revendication 2, **caractérisée en ce que** ladite base (3) a la forme d'une fourche ou d'un berceau ayant deux montures extérieures entre lesquelles est reçu l'élément de support (4), de manière à pouvoir tourner autour d'un axe passant à travers son centre géométrique (O).

5. Table de support de patient selon la revendication 2 ou 4, **caractérisée en ce que** sur la partie périphérique du secteur en arc de cercle (4) est enroulée une chaîne ou courroie dentée (10) qui est fixée au niveau de ses deux extrémités sur ledit secteur, et engrène partiellement avec un pignon (9) qui est entraîné par ledit engrenage réducteur (8), des moyens (10a, 10b) étant fournis pour garantir que le trajet de la chaîne ou courroie dentée (10) est fermé de manière aussi stricte que possible sur la périphérie du secteur (4) au niveau des deux côtés du pignon (9).

6. Table de support de patient selon les revendications 2 et 3, **caractérisée en ce qu'**elle comporte des moyens de couplage mutuel automatique entre les deux engrenages réducteurs (8, 11) pour faire en sorte que leurs fonctionnements simultanés donnent naissance à des rotations dans des directions opposées du secteur (4) autour du pivot (O), et de la structure (1) autour du pivot (O'), de sorte que le déplacement résultant de ladite structure est une translation dans le plan vertical sans inclinaison par rapport à l'horizontal.
